# EUROPEAN PATENT SPECIFICATION

(11) **EP 4 603 188 B1**
(45) Date of publication and mention of the grant of the patent: **13.05.2026**
(21) Application number: 25185351.1
(22) Date of filing: 15.03.2019
(51) Int. Cl.: G01N 33/48, G01N 33/483, G01N 33/487, G01N 33/49, G01N 11/10, G01N 11/00, G01N 11/12, G01N 33/86, B01L 3/00, G01N 35/10

(54) **COAGULATION TEST DEVICE, SYSTEM, AND METHOD OF USE**
KOAGULATIONSPRÜFVORRICHTUNG, SYSTEM UND VERFAHREN ZUR VERWENDUNG
DISPOSITIF DE TEST DE COAGULATION, SYSTÈME ET PROCÉDÉ D'UTILISATION

(43) Date of publication of application: 20.08.2025
(62) Divisional of application: 19919770.8
(73) Proprietor: Coagulation Sciences, LLC, Riverdale, NY 10471 (US)
(72) Inventor: GOLDSTEIN, Sheldon, New York, 10471 (US); KAGAN, Michael, New York, 10471 (US); LAUDER, Nicholas, New York, 10471 (US); COSMAN, Maury D., New York, 10471 (US)
(74) Representative: Marks & Clerk LLP

(56) References cited:
- WO-A1-2009/073851
- WO-A1-2015/184433
- US-A1- 2014 315 228
- US-A9- 2008 206 880

## Description

### BACKGROUND

Most existing coagulation tests, both point-of-care (POC) and laboratory based, diagnose bleeding abnormalities, but provide insufficient information regarding the underlying cause of bleeding. Current management of bleeding patients often includes use of "Massive Transfusion Protocols" (MTP). This protocol-guided strategy manages all patients with a one-size fit all approach and without consideration of underlying etiologies of coagulopathy in each individual patient. Laboratory tests for factor levels or visco-elastic tests require time and skilled technical staff to execute.

### SUMMARY

The coagulation test device described herein requires little-to-no laboratory skills to operate and presents individualized, evidence-based results, which may aide in treatment decisions within 15 minutes from inserting the sample tube to obtaining results. Furthermore, there have been reported cases of treatment selection and administration, which have led to thromboembolic events. The device allows for the in-vitro testing of potential therapeutic agents, which can aid in determining the underlying cause of coagulopathy, and guard against the administration of agents that may induce thromboembolic events

US2014/315228 discloses a known system comprising a cartridge containing a fluid sample placed in a blood analyzer device, a ferromagnetic material such as a ferromagnetic washer residing within a blood clotting analysis chamber is raised through the fluid sample and allowed to drop. The analyzer device then detects and measures the time required for the ferromagnetic material to fall through the fluid sample. As the viscosity of the fluid changes (either increasing or decreasing), the fall time of the ferromagnetic material through the fluid sample will change correspondingly.

The coagulation test device described herein is an in vitro diagnostic point-of-care device for measuring clotting time and clot characteristics of a whole blood sample under different hemostatic conditions. Results of the test are used as an aid in management of patients with coagulopathy of unknown etiology in order to help the physician determine appropriate clinical action to arrest bleeding. Additionally, the device addresses a key problem in perioperative medicine and solves this problem by testing the effect of specific hemostatic therapeutic agents on whole blood clotting time in a bleeding patient.

In the existing state of clinical practice abnormal test results are typically addressed with an experience-based educated guess on the course of therapy to administer. The coagulation test device described herein provides personalized, clinical guidance to physicians regarding etiology of the patient's specific coagulopathy. The device simultaneously compares the effect of several hemostatic agents on whole blood clotting time and derives the underlying etiology, based on the measured responses across these hemostatic agents.

In accordance with independent claim 1, a device is disclosed for processing a cartridge containing a whole blood sample. The device comprises a recess for receiving the cartridge, a vacuum source coupled to the cartridge, an actuator linked to the cartridge to agitate the cartridge; and a controller, a plurality of sensors in communication with the controller, each sensor configured to be associated with a test chamber of a plurality of test chambers comprised in the cartridge, and a plurality of magnets, wherein each magnet is configured to be positioned adjacent to a test chamber of the plurality of test chambers comprised in the cartridge.

The controller is configured to activate the vacuum source to move the whole blood sample from a container into a plurality of channels in the cartridge and subsequently into a plurality of reagent chambers where the blood mixes with a reagent, and then through a plurality of serpentine-shaped channels to a plurality of test chambers, activate the actuator to agitate the cartridge, receive signals from a plurality of sensors, each sensor associated with one of the test chambers, where the signals are based on the presence of a spherical member within a magnetic field generated by a magnet positioned adjacent to each of the test chambers, determine whether coagulopathy is present in the whole blood for each test chamber, and output an indicator whether coagulopathy is present in the whole blood for each test chamber on a display.

Further embodiments are defined by the dependent claims.

Other aspects of the invention will become apparent by consideration of the detailed description and accompanying drawings.

### BRIEF DESCRIPTION OF THE DRAWINGS

FIG. 1 is a perspective view of a coagulation test device according to an embodiment.
FIG. 2 is a block diagram of the coagulation test device shown in FIG. 1.
FIG. 3 is a perspective view of the coagulation test device shown in FIG. 1 with a test cartridge and protective cover removed,
FIG. 4 is a perspective top view of a test cartridge with inserted sample tube used with the coagulation test device shown in FIG. 1.
FIG. 5A is a perspective view of fluidics pathways of the test cartridge shown in FIG. 4.
FIG. 5B is a rear perspective view of the test cartridge shown in FIG. 4 and showing hydrophobic membranes.
FIG. 6 is a perspective view of the coagulation test device with the test cartridge in place prior to testing.
FIG. 7 is an enlarged cross-sectional view of a test chamber and a spherical member in the test cartridge shown in FIG. 4.
FIG. 8 graphically illustrates the relationship between travel speed of a spherical member within the test chamber and the resulting shear rate applied to the blood sample in the test chamber.
FIG. 9 illustrates a difference in magnetic field line map.

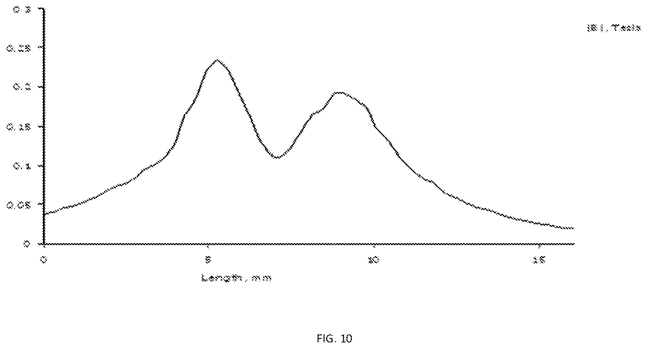

FIG. 10 graphically illustrates sensor detection of a spherical member within the test chamber.
FIG. 11 is a flow chart of a method of operation of the coagulation test device illustrated in FIG. 1.
FIG. 12 is a decision tree for determining cause of coagulopathy based on response to hemostatic reagents.
FIG. 13 graphically illustrates the sensor signals during the onset of clot formation.
FIG. 14 graphically illustrates detection of movement of the spherical member within the test chamber when a weak clot is present in the test chamber.

### DETAILED DESCRIPTION

Before any embodiments of the invention are explained in detail, it is to be understood that the invention is not limited in its application to the details of construction and the arrangement of components set forth in the following description or illustrated in the following drawings. The invention is capable of other embodiments and of being practiced or of being carried out in various ways.

One or more embodiments are described and illustrated in the following description and accompanying drawings. These embodiments are not limited to the specific details provided herein and may be modified in various ways. Furthermore, other embodiments may exist that are not described herein. Also, the functionality described herein as being performed by one component may be performed by multiple components in a distributed manner. Likewise, functionality performed by multiple components may be consolidated and performed by a single component. Similarly, a component described as performing particular functionality may also perform additional functionality not described herein. For example, a device or structure that is "configured" in a certain way is configured in at least that way, but may also be configured in ways that are not listed. Furthermore, some embodiments described herein may include one or more electronic processors configured to perform the described functionality by executing instructions stored in non-transitory, computer-readable medium. Similarly, embodiments described herein may be implemented as non-transitory, computer-readable medium storing instructions executable by one or more electronic processors to perform the described functionality. As used in the present application, "non-transitory computer-readable medium" comprises all computer-readable media but does not consist of a transitory, propagating signal. Accordingly, non-transitory computer-readable medium may include, for example, a hard disk, a CD-ROM, an optical storage device, a magnetic storage device, a ROM (Read Only Memory), a RAM (Random Access Memory), SIM card, register memory, a processor cache, or any combination thereof

In addition, the phraseology and terminology used herein is for the purpose of description and should not be regarded as limiting For example, the use of "including," "containing," "comprising," "having," and variations thereof herein is meant to encompass the items listed thereafter and equivalents thereof as well as additional items. The terms "connected" and "coupled" are used broadly and encompass both direct and indirect connecting and coupling. Further, "connected" and "coupled" are not restricted to physical or mechanical connections or couplings and can include electrical connections or couplings, whether direct or indirect. In addition, electronic communications and notifications may be performed using wired connections, wireless connections, or a combination thereof and may be transmitted directly or through one or more intermediary devices over various types of networks, communication channels, and connections. Moreover, relational terms such as first and second, top and bottom, and the like may be used herein solely to distinguish one entity or action from another entity or action without necessarily requiring or implying any actual such relationship or order between such entities or actions. Articles "a" and "an" are used herein to refer to one or to more than one (at least one) of the grammatical object of the article. By way of example, "an element" means at least one element and can include more than one element. Unless otherwise defined, all technical terms used herein have the same meaning as commonly understood by one of ordinary skill in the art to which this disclosure belongs.

Embodiments are described herein with reference to flowchart illustrations and/or block diagrams and/or figures. The flowchart, block diagrams and other illustrations in the present disclosure illustrate the architecture, functionality, and operation of possible implementations of systems, methods, computer program products (non-transitory computer-readable medium storing instructions executable one electronic processors, such as a microprocessor, to perform a set of functions), and the like according to various embodiments of the present invention. In this regard, each block in the flowchart or block diagram(s), or accompanying figures herein may represent a module, segment, or portion of instructions, which comprises one or more executable instructions for implementing the specified logical function(s). In some alternative implementations, the functions noted in the blocks or figures may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration and/or figures and combinations of blocks in the block diagrams and/or flowchart illustration and/or figures can be implemented by special purpose hardware-based systems that perform the specified functions or acts or carry out combinations of special purpose hardware and computer instructions.

Herein, various terms that are well understood by those of ordinary skill in the art are used. The intended meaning of these terms does not depart from the accepted meaning.

The terms anti-coagulant or anti-coagulating agent may be used interchangeably, and refer to compositions that are added to, or are present in biological specimens which inhibit natural or artificial coagulation, prolonging coagulation time. Examples of anti-coagulants include, but are not limited to, sodium citrate, hirudin, chelating agents, exemplified by ethylenediamine tetraacetic acid (EDTA), diethylenetriamine pentaacetic acid (DTPA), 1,2-diaminocyclohexane tetraacetic acid (DCTA), ethylenebis(oxyethylenenitrilo) tetraacetic acid (EGTA), or by complexing agents, such as heparin, and heparin species, such as heparin Sulfate and low-molecular weight heparins, as well as coumarins and indandiones, factor Xa inhibitors, and thrombin inhibitors.

The term coagulopathy as used herein refers to any bleeding disorder that affects the way a patient's blood clots. The term hyper-coagulable as used herein refers to a state of abnormal increase towards blood clot formation. The term hypo-coagulable as used herein refers to a state of abnormal decrease away from blood clot formation.

Examples of excitation sources as used herein may be magnetic fields, electromagnetic fields, light, or ultrasonic energy. Excitation sensors as used herein are means capable of detecting the presence, absence, or changes in excitation source as affected or disrupted by the test element in the test sample.

The term blood clot lysis and fibrinolysis may be used interchangeably and as used herein refers to the breakdown of fibrin, usually by the enzymatic action of plasmin. The term clot integrity and clot strength may be used interchangeably and as used herein refers to the strength of a clot that has formed as a result of a fibrin and platelets mesh. The term premature thrombolysis as used herein refers to the premature dissolution of a clot after formation and is indicative of a defect in hemostasis.

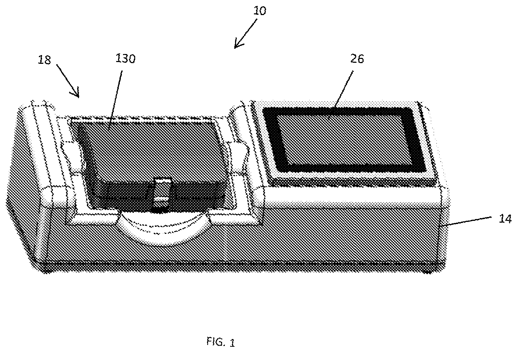

FIGS. 1-2 illustrate a coagulation test device 10 according to some embodiments. The coagulation test device 10 provides the ability to simultaneously evaluate clotting time and determine clot characteristics of whole blood under multiple hemostatic conditions according to some embodiments. As illustrated in FIG. 1, the coagulation test device 10 includes a housing 14, a recessed area 18 configured to receive a cartridge 30, and a display 26. As shown, the recessed area 18 and the display 26 are positioned adjacent to one another in the housing 14; however other configurations and orientations between the recessed area 18 and display 26 are possible.

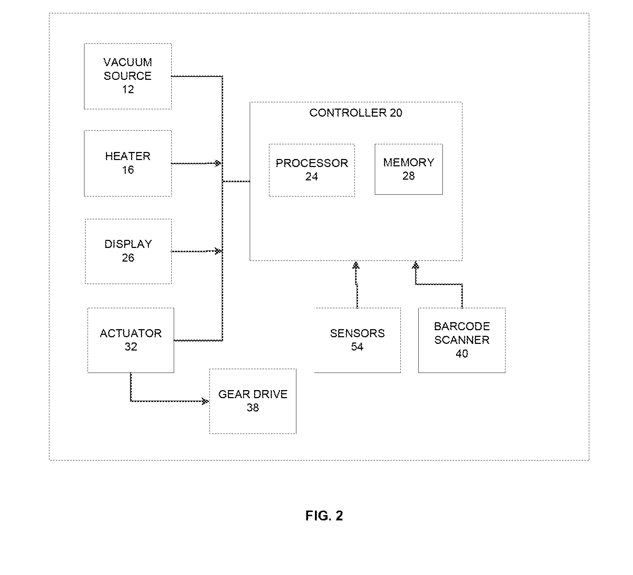

With reference to FIG. 2, the coagulation test device 10 also includes a vacuum source 12 supported by the housing 14 and a heater 16 supported by the housing 14. The coagulation test device 10 further includes a controller 20 including an electronic processor 24 and a computer-readable, non-transitory memory 28. The memory 28 stores instructions that are executed by the electronic processor 24 to provide the functionality of the controller 20 as discussed herein. The controller 20 is also coupled to the display 26 and is configured to output graphical information and data. For example, in some implementations, the device 10 is configured to output on the display 26 specific data related to hemostatic reagents under test and whole blood clotting time for each channel while the test is in progress. Hemostatic agents may include fibrinogen, Factor VIII, Factor IX, cryoprecipitate, human plasma, Factor VIII and von Willebrand, 3 factor Prothrombin Complex Concentrate (PCC), 4 factor Prothrombin Complex Concentrate (PCC), Protamine Sulfate, platelets, Heparinase, Factor VII, Factor VIIa, and Factor XIII, however other suitable hemostatic agents may be employed. When the test is completed, the display 26 also can display a diagnosis and other testing characteristics derived from analysis of the test channel data. The controller 20 is also coupled to the vacuum source 12 and the heater 16 to control activation and deactivation.

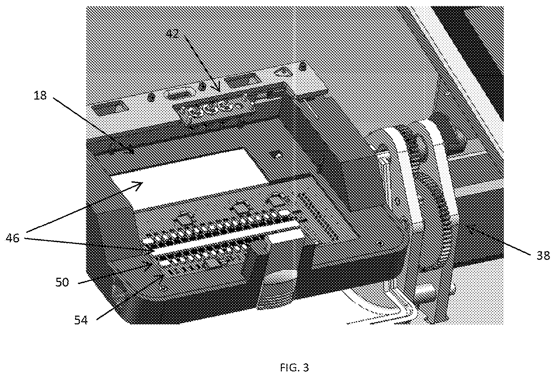

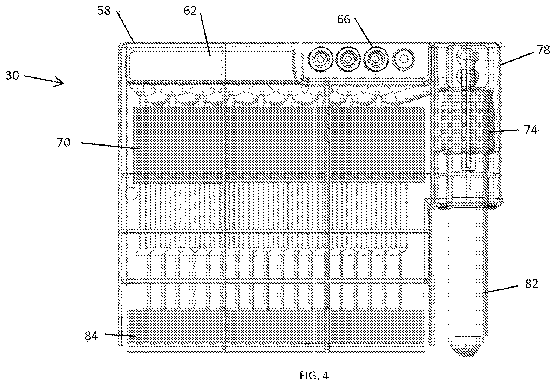

With reference to FIG. 3, the recessed area 18 is configured to receive the cartridge 30, which allows for up to 18 channels of clotting time testing in a single cartridge 30. In one configuration, the cartridge 30 is about the size of a microtiter plate (e.g., 9.7 mm by 11.8 mm) and conducts 18 individual coagulation tests. Additional or fewer channels may be utilized within the envelope of the cartridge 30. For example, for purposes of illustration, FIGS. 4-5B show 18 channels in the cartridge 30, however the cartridge 30 may include more or fewer than 18 channels. In one embodiment, the device 10 accepts a sample tube containing 4.5 ml of whole blood. For 18 channels of coagulation testing, this embodiment creates 18 aliquots of approximately 150 microliters for testing. The construction and dimensions of the cartridge 30 may be altered to reduce the sample volume to 2.7 ml by reducing the size of the reagent chambers and subsequent test chambers.

With continued reference to FIG. 3, the recessed area 18 is mechanically linked to a gear drive 38 for agitating the cartridge 30 and samples therein. The gear drive 38 is in communication with the controller 20 via an actuator 32 (e.g., motor, pump, etc.). In one example, the actuator provides an agitation/rocking speed between 90 degrees and 180 degrees per second with a rocking angle between 10 degrees and 75 degrees. One cycle duration is between 1 and 5 seconds. In other examples, the actuator can provide a suitable rocking speed and rocking angle that may be less than or greater than the parameters provided herein. Similarly, the cycle duration can be suitably adjusted within the scope of the operation of the device 10.

The recessed area 18 also includes one or more vacuum ports 42 that interface with the cartridge 30, one or more heater regions 46 for incubation and test regions, a plurality of magnets 50, and a plurality of sensors 54 in communication with the controller 20.

FIG. 4 illustrates an embodiment of the cartridge 30. The cartridge 30 includes a housing 58, a waste collection area 62, and one or more vacuum ports 66 configured to interface with the one or more vacuum ports 42 on the recessed area 18. The cartridge 30 also includes a reagent vacuum region 70, a sample tube interface 74, a sample tube housing 78 configured to receive a sample tube 82 (as shown in the sample tube housing 78 in FIG. 4), and a test channel vacuum region 84.

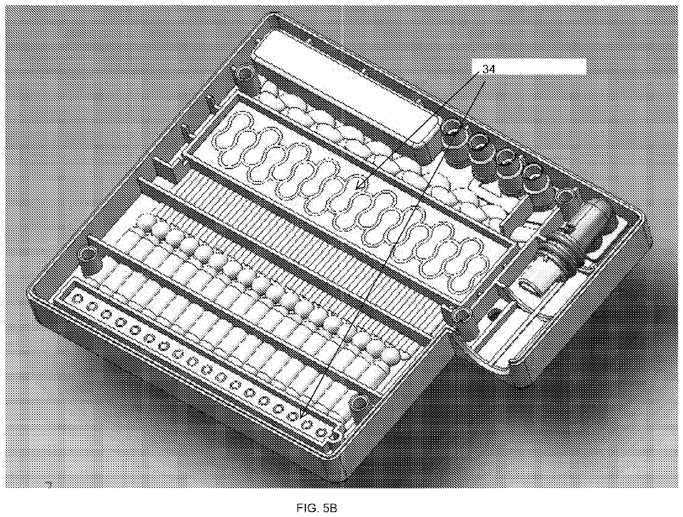

The cartridge 30 is designed for injection molding and secondary assembly operations. The cartridge 30 is sealed with a film that is compatible with blood and does not influence coagulation. The cartridge 30 includes a plurality of chambers that are isolated from each other. The blood sample is introduced into the cartridge 30 by the vacuum source 12. Hydrophobic filters 34 (FIG. 5B) are used to stop the flow of blood when it has been fully aspirated. The vacuum ports 66 are at atmospheric pressure when the cartridge 30 is outside of the device 10. Since no pressure is being applied to the sample, the sample remains in the sample tube 82 until it is loaded onto the device 10 and the sample sequence has begun.

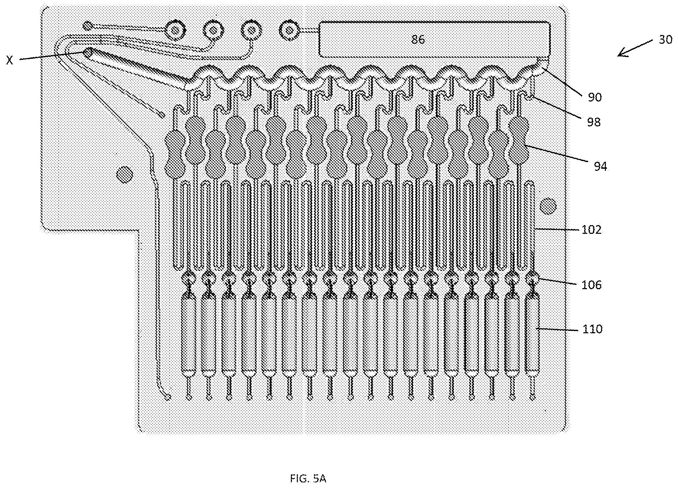

The cartridge 30 includes a common feed channel X (shown in FIG. 5A) that connects the sample input to the chambers containing the individual hemostatic reagents. Each reagent chamber is connected to one of the vacuum ports 66 through a series of hydrophobic filters 34 for each channel. As the sample is aspirated into the cartridge 30, each channel is filled sequentially at a rate established by a predetermined vacuum pressure of the vacuum source 12. When an individual channel is filled completely, the hydrophobic filter 34 shown in FIG. 5B for that channel is blocked and the channel stops filling beyond its capacity. The remaining channels fill until each of the filters are blocked. The controller 20 monitors duration of applied vacuum and may also monitor the pressure drop across the filters to determine when all of the channels are filled.

The fluidics of the cartridge 30 are further described and illustrated in FIGS. 5A-B according to some embodiments. The cartridge 30 includes a waste region 86, a sample feed channel 90 in communication with the waste region 86, and one or more hemostatic reagent chambers 94 in communication with the sample feed channel 90 via respective channels 98. The cartridge 30 also includes one or more serpentine mixing channels 102 at an outlet of a respective hemostatic reagent chamber 94. The serpentine mixing channels 102 are in fluid communication with one or more anticoagulant reversal chambers 106 (for if an anti-coagulant was used to anti-coagulate the whole blood sample), respectively, which are each then in respective communication with one or more clotting test chambers 110. As noted above, and as illustrated herein, the cartridge 30 is shown with 18 individual test channels 112, with each channel including a reagent chamber 94, a serpentine fluid path 102, and a test chamber 110, however, the cartridge 30 may include more or fewer than 18 test channels 112 in other constructions.

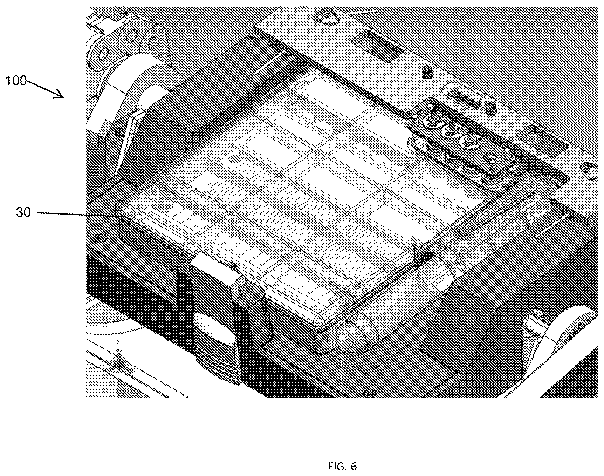

FIG. 6 illustrates a system for determining clotting characteristics of a whole blood sample 100 including the device 10 and the cartridge 30. In particular, the cartridge 30 is shown in position in the recessed area 18 of the device 10. The vacuum ports 42 in the recessed area 18 are in communication with the vacuum ports 66 on the cartridge 30.

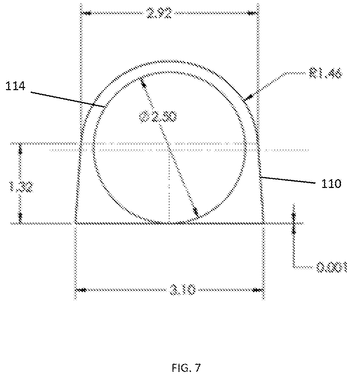

Clot formation in whole blood or plasma can be measured in many different ways. Electrical conductivity requires contact with the whole blood. Capacitive measurements do not require contact and can be measured through barrier films; however, these techniques require some type of electrical connection to the testing device in order to bring signals in and out of the device. The coagulation test device 10 described herein utilizes a non-contact measurement method where a sphere 114, comprised of a magnetic material, is within each of the 18 test chambers 110 and is used to determine the viscosity of the whole blood in each test chamber 110. FIG. 7 illustrates a cross-section of an enlarged test chamber 110 and the spherical member 114 positioned therein. Agitating the test chamber 110 causes the spherical members 114 to roll through the whole blood in each test chamber 110. The sensors 54 are positioned in close proximity to the test chamber 110 to measure the presence of the spherical member 114 in the test chamber 110 as it is agitated by the device 10. In one construction, the test chambers 110 have a width of 3.0 mm and a length of 18 mm to accommodate a sample aliquot volume of approximately 150 microliters. In other constructions, the test chambers 110 could have a width of 1.5 mm and a length of 10 mm in order to reduce the sample aliquot volume to approximately 70 microliters. In one example construction, the test chambers 110 are spaced 4.5mm apart which allows for 18 channels of clotting time testing to be incorporated into the envelope of the cartridge 30. Other suitable dimensions are possible within the scope of the device described herein.

Sensing travel speed within a test chamber 110 is accomplished by measuring the change in magnetic flux as the spherical member 114 passes in proximity to the sensors 54 located along the path of each test chamber 110. The sensor 54 does not require contact with the test chamber 110 and can be spaced up to 0.8 mm away from the test chamber surface. The sensors 54 sense the signals generated within the housing 58 of the cartridge 30 from their respective test chamber 110. This non-contact method of measurement enables complete separation between the cartridge 30 and the device 10, thereby eliminating the need for the device 10 to make contact with the whole blood sample. This configuration also eliminates any device cleaning or maintenance steps between test samples. It further eliminates the possibility of the device losing functionality due to a clot in the device fluid pathways.

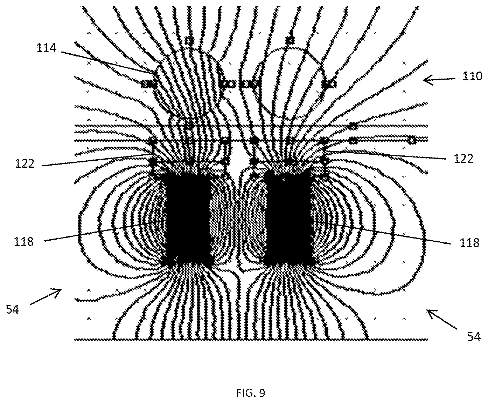

Each test chamber 110 is associated with two sensors 54, located linearly along the travel path of the spherical member 114 in the test chamber spaced apart by a distance (e.g., 9.5 mm). With reference to FIG.9, each sensor 54 is comprised of a magnetic member 118 (e.g., comprised of one or more rare earth metals) of sufficient magnetic field strength whereby the magnetic field extends into the test chamber region, and a Hall-Effect sensor 122, which is located between the magnetic member 118 and the test chamber 110. The Hall-Effect sensors 122 produce a voltage signal proportional to the magnetic field of between 8 and 10 millivolts per gauss. The controller 20 records a baseline, quiescent reading of the magnetic field for each sensor 54 at the beginning of an agitation cycle. This baseline measurement is used to establish the amount of disturbance of the field as the spherical member 114 passes over the sensors 54. As the spherical member 114 passes over the sensors 54, the spherical member 114 causes a disturbance in the quiescent magnetic field and is detected by the sensor 54. This disturbance in magnetic flux is detected by the Hall-Effect sensor 122 and converted to a voltage. The voltage signal for each sensor 54 is then transmitted to the controller 20. A signal threshold is established to remove signal artifacts. The voltage signal form the sensor 54 can be converted to a digital signal via a series of analog to digital converters. Since there are two sensors 54 located for each test chamber 110, the time between the peak of the disturbances of the two sensors 54 relates directly to the travel time of the spherical member 114 within the test chamber 110. By this method, the viscosity of the fluid in the test chamber 110 can be traced as the device 10 agitates the test chamber 110 over a period of time, causing the spherical member 114 to pass by the sensors 54. FIG. 9 illustrates a magnetic field map showing the interaction with the test chamber 110 and the spherical member 114. As shown in FIG. 9, the map on the left shows how the spherical member causes more field lines than the map on the right, where no spherical member is present,

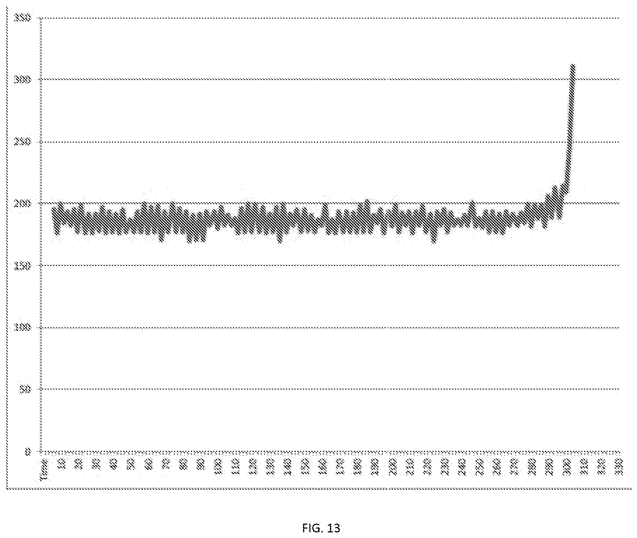

The test chambers 110, sensors 54, and controller 20 determine the travel time of the spherical member 114 in each test chamber at a predetermined and programmable agitation rate. The viscosity of the fluid in each test chamber 110 is proportional to the travel time within the chamber. As such, the progression of clotting can be viewed as an increase in fluid viscosity vs. time. The coagulation cascade is truly a cascade of events and is therefore non-linear The traces of travel time for the device 10 can detect and monitor quiescent blood status as it approaches coagulation. Shortly after this initial onset of clotting, the fluid rapidly approaches a high viscosity by forming a clot as shown in FIG. 13. The angle of trajectory from a fluid state to clot formation is also measured and used as a basis for diagnosis of apparent factor or platelet function deficiency.

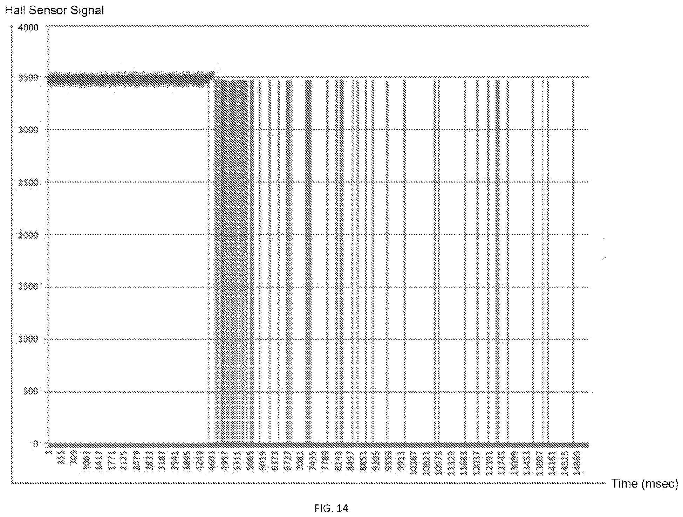

In addition to clotting time, the device 10 is capable of determining the integrity or strength of the clots being formed. Each test chamber 110 within the device 10 has two independent sensors 54 (as described above), by which travel time is calculated. When the spherical member 114 fails to traverse both sensors 54 within a test chamber, a travel time cannot be calculated. This is indicative of a clot that is impeding the full travel of the spherical member 114 within a test chamber 110. However, since the gravitational forces on the spherical member 114 within a test chamber are less than 1G, it is possible for a spherical member 114 to be held by a weak clot, where the spherical member 114 will continue to move over a limited range of the full chamber length. The device 10 monitors the signals from both sensors 54 and may detect that one of the two sensors 54 is continuing to provide a signal. This signal is indicative of a weak clot that is preventing the spherical member 114 from fully traveling the length of the test chamber 110, yet is still allowing the spherical member 114 to move. As shown in FIG. 14, only one of the two sensors continues to show movement of the spherical member 114.

FIG. 14 shows a condition where the spherical member 114 is transitioning across only one of the sensors 54 with each agitation or duty cycle of the cartridge, indicating a weak clot. The frequency by which the spherical member 114 is detected by a single one of the two sensors 54 is indicative of clot integrity, where a high frequency of signal generation indicates a weak clot. As the number of single sensor signals decreases, this is indicative of increased clot strength. No movement of the spherical member 114 would indicate a strong clot after clot formation, yielding a frequency of zero or below a threshold set by the device 10. The device 10 establishes a cut-off value for clot integrity.

In one example, the duty cycle of the spherical member 114 is proportional to the integrity of the clot where the duty cycle of greater that 40% is indicative of a severely weak clot, the duty cycle of between 25% and 40% is indicative of a moderately weak clot, the duty cycle of between 10% and 25% is indicative of a moderately strong clot, and the duty cycle between 0%) and 10% is indicative of a strong clot.

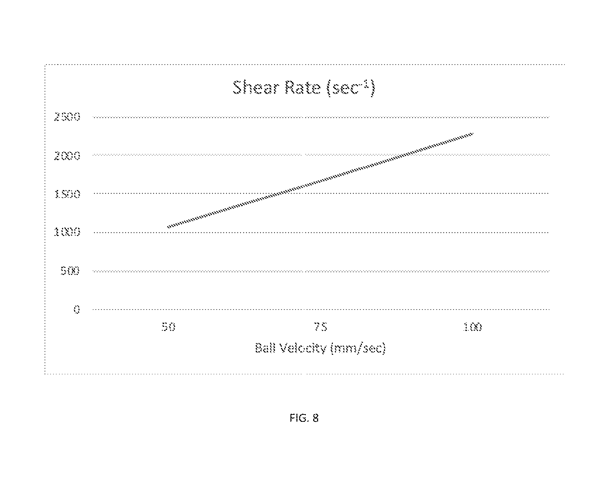

The relative size of the spherical member 114 within the test chamber and the test chamber diameter helps to induce coagulation in a manner similar to physiological clotting mechanisms. It is known that shear stress within the human vascular system promotes coagulation, Many coagulation test systems require the addition of a high surface area pro-coagulant such as Celite or Kaolin to reduce the normal clotting time to a level that meets the needs for a rapid clotting time. The method and apparatus described herein eliminates the need for pro-coagulants by inducing physiological shear rate and shear stress within each test channel of between 500 and 4,000 sec⁻¹ or 100 to 1,000 dynes per second. FIG. 8 illustrates the relationship between the travel speed of the spherical member 114 within the test chamber 110 and the resulting shear rate, based upon the diameter of the test chamber and the relative size of the spherical member 114 in the test chamber. The shear rate can be adjusted by altering the agitation angle, the test chamber diameter and the relative spherical member diameter. However, the system could also be used with the addition of a pro-coagulant, such as kaolin, citrate, tissue factor, phospholipid, or other appropriate activator, if a more rapid time to test results is desired.

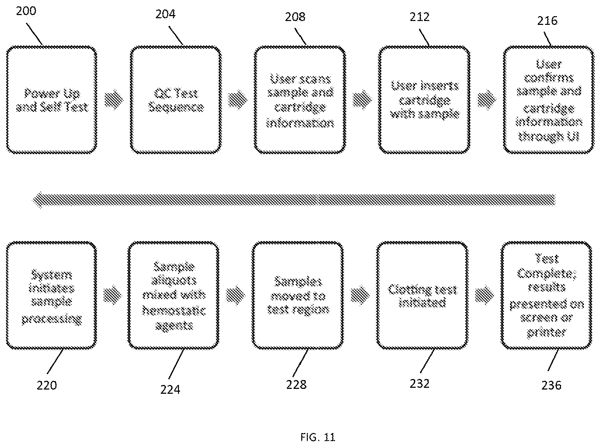

With reference to FIG. 11, sample processing begins with power up of the device 10 (at 200). The device 10 undergoes quality control tests (at 204). The device 10 performs a quality check to ensure that all electro-mechanical subsystems are in proper working order. Subsystems such as the vacuum system, the temperature elements and magnetic sensors are all checked prior to allowing a user to initiate a sample test. Additionally, the device is supplied with a re-usable QC cartridge which agitates the QC cartridge in a manner similar to a sample test to ensure that all electro-mechanical systems are working properly. The re-tisable cartridge test results are recorded in the controller or memory. The QC cartridge is removed and the device is then ready to accept a patient sample. Next, the sample tube (filled with whole blood from a patient; the sample tube includes a unique barcode) is identified to the device 10 (at 208). The device 10 allows for entry of all information required to run a test with no operator typing. The device 10 can include a barcode scanner 40 in communication with the controller 20. The operator places the sample tube barcode in front of the scanner 40. The scanner 40 records the barcode and sample information into the memory 28 or other storage device (e.g., database, local or remote from device 10). The device 10 has an override function that can permit manual entry of patient information via the display 26 (e.g., touchscreen). The patient sample also must be associated with a test cartridge 30. The operator places the barcode affixed to the cartridge in front of the scanner 40. The scanner 40 records the cartridge 30 information into the memory 28 or other storage device (e.g, database, local or remote from device 10). If the sample tube does not have a barcode, then the operator can be prompted to enter the information via an on-screen keyboard on the display 26,

The sample and cartridge 30 are loaded onto the device 10 (at 212) and the operator confirms (at 216) the sample and cartridge 30 via a user interface on the display 26. A cover 130 on the device is lowered onto the recessed area 18. The cover 130 includes the vacuum connections that interface with the vacuum ports 66 on the cartridge 30 necessary for moving fluids through the cartridge 30 during sample processing and prior to initiating the clotting time tests. The vacuum ports 66 on the cartridge 30 are connected to the vacuum ports 42 in the device 10 through the action of closing the cover 130. The cover 130 also applies pressure to the cartridge 30 to maintain a uniform distance between the cartridge 30 and the sensors 54 within the device 10. The operator initiates the testing sequence via the user interface on the display 26.

Samples presented to the device 10 are typically anti-coagulated in order to allow time between sample collection and sample testing. This is a common practice for most blood testing procedures. The device 10 also can test a sample that is not anti-coagulated with a cartridge that does not contain the anti-coagulant reversal agent (e.g., calcium); however, there are time constraints for placing a non-anticoagulated sample into the cartridge 30 and onto the device 10.

The cartridge 30 is configured to receive a sample vacuum drawtube (e.g., an evacuated container with a flexible cap) as a sample input device in the sample tube housing 78. This eliminates the need for the sample to be pipetted into the cartridge 30, a process common to laboratory instruments, but not required for the device 10. The operator inserts the sample drawtube directly into the cartridge 30 (in the sample tube housing 78) and places the cartridge 30 onto the recessed area 18 in the device 10. The cartridge 30 prevents the blood sample from moving into the cartridge by controlling any vent path from being opened prior to placement on the device 10. The sample in the tube is accessed by one or more needles (e.g., two needles) that pierce the flexible cap. The operator inserts the sample tube into the sample tube receiver on the cartridge. Gentle insertion force causes the needle assembly to pierce the sample tube cap. One needle serves to aspirate the sample and the other serves as a vent to allow the blood to flow from the drawtube when the vacuum is applied.

The device 10 processes (at 220) the sample with a known set of programmed parameters. The first step is to pierce the flexible cap to draw the blood from the sample tube and fill the sample feed channel 90 and the connected reagent-containing chambers 94. The blood travels from the sample tube into the cartridge 30 at point X (shown in FIG. 5A) and fills the sample feed channel 90, and begins to fill the reagent-containing chambers 94. The controller 20 activates the vacuum source 12 in the housing 14 to apply vacuum to the blood sample in the cartridge. A pressure regulator that is in line with the vacuum pump is read by the device 10 so that the pressure is controlled to a preset value. Typical vacuum levels for sample aspiration are between 50 and 100 millibars (mb). In this sequence, the initial sample is now split into 18 equal and isolated aliquots of the blood sample.

The controller 20 selects certain valves in the vacuum ports 66 thereby controlling the direction of the vacuum. The blood sample continues to move through the various channels in the cartridge 30. The reagent chambers 94 contain individual doses of a variety of hemostatic agents. These agents are dried and remain in the reagent chamber 94 as part of the manufacturing process. Drying methods may be lyophilization or air drying, depending upon the reagent properties. The design of the cartridge is such that the blood sample is drawn into the reagent chambers 94 from the bottom of the chamber and drawn to the upper portion of the chamber by the vacuum source 12. The dried reagents are rehydrated by the entering sample volume of blood (at 224). Each reagent chamber 94 contains a hydrophobic filter, which prevents the reagent chambers from overfilling. The device 10 applies vacuum for a pre-set time duration or monitors the pressure drop across the hydrophobic filters until all filters are blocked.

Each sample-reagent complex must be separated from its nearest neighboring sample-reagent complex in order to avoid cross contamination. After filling the individual reagent chambers 94, the device 10 clears the sample feed channel 90 that connects the reagent chambers 94 by closing valves used to direct the sample to the reagent chambers 94 and opens valves to direct the sample feed channel 90 contents to the waste area 62 in the cartridge 30. An absorbent material collects the waste from the feed channel 90. Each sample aliquot is now isolated from its adjacent reagent chambers 94 by a large air gap created by the empty feed channel 90.

Coagulation is a temperature dependent phenomenon. The controller 20 activates the heater 16 to provide thermal energy (via heater region 36 on the recessed area 18) to the cartridge 30 and bring the sample aliquots within the reagent chambers 94 to a programmable temperature value of between 25 and 40 degrees Celsius with normal test temperature of 37 degrees Celsius. This range allows for testing samples under normal, hypo-thermic and hyperthermic conditions. The sample aliquots are incubated with the hemostatic agents for a programmable period of time, typically between 1 minute and 10 minutes. In other embodiments, the sample aliquots are incubated with the hemostatic agents between 3-5 minutes. This allows the temperature of the sample aliquots to equilibrate to the proper temperature as well as allowing the hemostatic agents to fully dissolve and diffuse into the sample aliquots.

Up until this point, the blood sample and the reagents are anti-coagulated to prevent the blood sample from starting to clot until all sample aliquots are ready to be tested. Prior to initiating the clotting time test for each test channel, the anti-coagulant must be reversed. The serpentine-shaped channel 102 connects the reagent chambers 94 with the test chambers 110. The narrow diameter of the serpentine section increases the fluid velocity as it travels from the reagent region to the test region. In line with the flow of each channel is a precise amount of dried or lyophilized calcium. The traveling fluid rehydrates the calcium and mixes with the sample aliquots at the time of transfer. The cartridge 30 contains individual quantities of calcium in each channel, directly before the sample enters the test region. The calcium mixes with the aliquots as it proceeds to the test region. The anticoagulant in each aliquot has now been reversed and the clotting cascade can begin.

The controller 20 transmits a signal to change the direction of the vacuum source 12 via the valves and directs the flow of the sample aliquots from the reagent chambers 94 to the test chambers 110 (at 228). Typical vacuum levels are between 50mb and 100mb. The cartridge 30 and test chambers 110 are placed at a predetermined angle between 10 and 40 degrees relative to the horizontal position of the cartridge when placed on the device so as to facilitate channel filling and to minimize the possibility of trapping an air bubble in the test chambers 110. Each test chamber 110 has a hydrophobic filter in line with the vacuum source 12 to prevent overfilling of the test chamber and drawing blood into the device vacuum system.

With all (or some) of the test chambers 110 filled and the sample aliquots' anticoagulation state reversed, the controller 20 activates the actuator 32 to begin (at 232) to agitate the cartridge 30 about a central axis, centered on the test channels 110 and to begin the clotting test. This action causes the spherical members 114 within the cartridge 30 to roll from one end of the test channel to the other end in a uniform manner. With each agitation cycle, the spherical members 114 in each test channel pass over the sensors 54 associated with each channel 110 to generate a signal that is proportional to the viscosity of the blood sample within each test channel 110. The sensors 54 transmit the generated signals to the controller 20.

As noted above, the device 10 utilizes a non-contact method of detecting the motion and travel time of the spherical members 114 within the cartridge channels. The current method utilizes the magnetic properties of the 400 series stainless steel ball; however, other non-contact detection methods may be used, such as ultrasonic, electromagnetic, optical, etc.

Each sensor 54 includes a pair of neodymium-iran-boron rare earth magnets 118 and two Hall-Effect sensors 122 for each channel 110. Each magnet 118, separated by approximately 9.5 mm along the linear path of each test channel creates a localized magnetic field through the Hall-Effect sensor 122 and into the specific region of the cartridge 30. When the magnetic stainless steel ball passes through the magnetic field, the Hall-Effect sensor 122 detects the change in flux above a quiescent baseline signal caused by the presence of magnetic ball and generates a voltage. The device measures the baseline (quiescent) magnetic signal at the beginning of each cycle. The voltage from each sensor 54 (if there is a voltage detected) is transmitted to the controller 20 for further processing.

Other excitation sources and sensor technologies could be employed to measure the coagulation effect in each channel; however, they may be subject to channel-to-channel crosstalk. Ultrasonic sensors for each channel could also be used. In this case, the spherical members 114 in each channel would not be required to be magnetic, but of a density much greater than whole blood, so that the ultrasonic reflection would be large enough to receive a signal. Electromagnetic sensors, similar to miniature metal detectors, could also be used. In this case, the spherical members would need to be electrically conductive, but not magnetic. Optical detection of the spherical members could be used. In this case, reflective sensor elements that provide an excitation light source and an adjacent light detector would detect the reflection of the spherical members as they pass over the optical sensor. The advantage of the use of magnetic sensing is that the magnetic fields between channels are self-isolating, due to the fact that they are all of the same polarity and do not interfere with adjacent channels as close at 4mm apart.

During the first minutes of the testing, the baseline or normal viscosity of the sample aliquots is established. As fibrin begins to form in each of the test channels, an increase in viscosity of between 10% and 20% is observed and recorded by the controller 20. Shortly after this increase, the coagulation cascade progresses rapidly to the point where the viscosity of the sample in a given channel is greater than the spherical member's ability to travel through the sample. The sensors 54 in line with the specific channel 110 sense that there is no longer a voltage being generated. The controller 20 interprets this as clot formation and records the time of the clot. The test proceeds until all channels have clotted or the pre-programmed maximum test time has been achieved (e.g., the test is complete at 236).

As described earlier, clot integrity or strength is also considered when considering clotting time and the determination of a complete clot. Only those clotting times that are associated with a firm clot are considered in determining the source of coagulopathy. Physical observation of weak clots show small fibers formed around the spherical member that inhibit the sphere from traversing the entire length of the test channel; however, the spherical member is able to move a short distance and across one of the two sensors in the test channel. By contrast, a high integrity clot captures the spherical member completely and allows for little or no travel after clot formation.

After clot formation, the sensor signals can assess clot integrity. Clot formation is determined when the sensors 54 no longer see the two sensor peaks in each device agitation cycle. In a clot of high integrity/strength, neither sensor 54 produces a signal after clot formation. In a weak clot of low integrity, one of the sensors 54 continues to produce a signal, indicating that the clot is allowing the spherical member 114 to travel over a limited distance. Clot integrity is quantified by looking at the average signal in the channel from the time the clot is initially formed, when at least one sensor reports no signal, until the end of the programmable testing period of between 300 and 1,800 seconds. The lower the number, the higher the clot integrity. A high integrity clot gives a value of between zero and 100. A moderate integrity clot gives a value between 101 and 400. Low integrity clots yield clot strength values greater than 401 and as high as 2,000.

The device 10 compares the clotting times and clot integrity to two (2) untreated or reference channels within the cartridge 30 to the therapy containing channels. Other clotting tests determine clotting times in seconds and compare to an established range of clotting times. The device 10 looks at clotting times relative to the reference sample channels, in the form of a clotting time ratio, as a way of determining the response to the various hemostatic agents. A value of 1.0 indicates no difference between the hemostatic agent and reference sample channels. A value below 1.0 indicates a reduction in clotting time as compared to the reference channels and a value greater than 1.0 indicates a prolongation of clotting time. The coefficient of variation (CV) in clotting times may be as high as 12%, so the threshold for a 'response' to a reagent is based on a reduction greater than the CV. A reduction in clotting time, normalized to the reference channels, of greater than 20% with a clot of high integrity (equal to or less than 100), is considered a valid response to the reagents within a specific channel. If the clotting time for a test chamber is below the lower limit of an established normal range (typically between 120 and 270 seconds), the chamber results are flagged as potentially hypercoagulable. If the clotting time is above the upper limit of the established normal range, the test chamber is flagged as hypocoagulable.

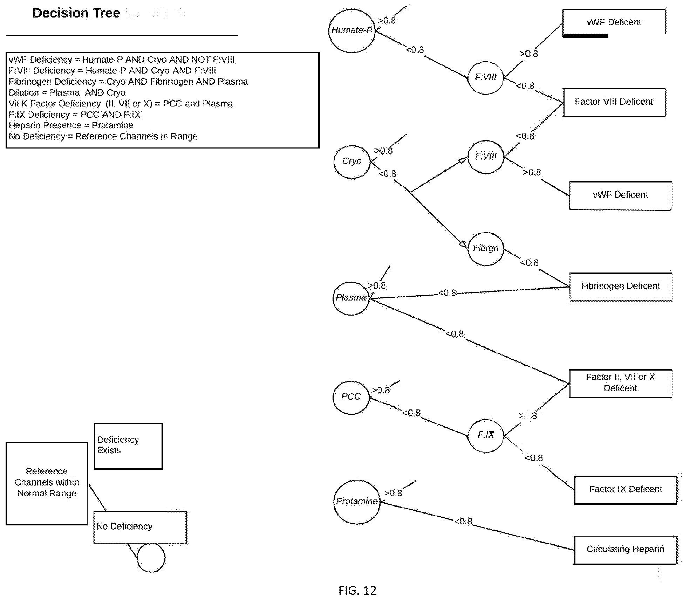

The etiology of coagulopathy is determined by the clotting time ratios described and clot strength of each test chamber 110 as compared to reference sample aliquots included within the cartridge 30. Information for hemostatic agents that reduce clotting time as well as hemostatic agents that do not reduce clotting time are combined to isolate either a specific etiology or a probable group of factors that are deficient and thereby causing the coagulopathy. For example, with reference to the decision tree in FIG. 12, if a cartridge 30 and sample responds with reduced clotting time (indicated by the <0.8 ratio) in the cryoprecipitate channels, but does not respond in the Factor VIII channels (indicated by the >0.8 ratio), then the deficiency is likely to be von Willebrand Factor or fibrinogen, since cryoprecipitate contains all three coagulation factors. Likewise, if the sample responds to the fibrinogen channels and the cryoprecipitate channels but does not respond to the Factor VIII or Factor VIII/vWF complex channels, then the determination is likely to be a fibrinogen deficiency.

These responses to the various hemostatic agents create patterns or signatures that are indicative of specific conditions that are reported at the end of the testing sequence. The display 26 can provide a visual indication of each channel's clotting time and the ratio to the untreated reference channels. The display 26 also can provide the resulting deficiencies or diagnosis, subject to interpretation by a physician, regarding the possible cause of the coagulopathy, based upon clotting times, clotting ratios and clot strength across all test channels. The analysis performed by the controller is multi-variate in nature, looking at all channel data in determining the etiology of coagulopathy. The device 10 may also include a printer to print the information and data provided on the display 26. Response to therapy is established by a threshold of the ratios to the reference channels. Since there is inherent variation across channels of up to 12%, the thresholds are established to take this into account. Response to a hemostatic reagent is defined as a ratio greater than a predetermined threshold (e.g.,a change of 20% or more as compared to a reference channel).

The device 10 may also be used in testing how a clot breaks up after clot formation has been established. Normally, there is a process called fibrinolysis where a blood clot dissolves naturally. In device 10 described herein, the cartridge 30 could continue to agitate for about 30-60 minutes while monitoring for the spherical members 114 to resume movement. While this should not occur in a normal sample, -some patients, for example traum a patients may experience hyperfibrinolysis, where the clots dissolve too quickly and bleeding starts up again.

Initial agitation cycles of between 1 and 2 seconds allow sufficient time for the spherical members 114 to traverse from one end of the cartridge 30 to the other. As clot formation begins, the viscosity of the sample aliquot increases, causing the travel time to increase. The device has the ability to apply an adaptive approach to the agitation cycle parameters.

## Claims

1. A device (10) for processing a cartridge (30) containing a whole blood sample, the device comprising:
a recess (18) for receiving the cartridge,
a vacuum source (12) configured to be coupled to the cartridge,
an actuator (32) configured to be linked to the cartridge to agitate the cartridge,
a controller (20),
a plurality of sensors (54) in communication with the controller, each sensor configured to be associated with a test chamber of a plurality of test chambers comprised in the cartridge, and
a plurality of magnets (50, 118), wherein each magnet is configured to be positioned adjacent to a test chamber of the plurality of test chambers comprised in the cartridge;
wherein, in use, the controller is configured to:
activate the vacuum source to move the whole blood sample from a container into a plurality of channels in the cartridge and subsequently into a plurality of reagent chambers in the cartridge where the blood mixes with a reagent, and then through a plurality of serpentine-shaped channels to a plurality of test chambers in the cartridge, each of the plurality of test chambers comprising a spherical member,
activate the actuator to agitate the cartridge,
receive signals from the plurality of sensors, each sensor associated with a test chamber, wherein the signals are based on the presence of the spherical member in a respective test chamber within a magnetic field generated by a magnet of the plurality of magnets,
determine whether coagulopathy is present in the whole blood for each test chamber, and
output an indicator whether coagulopathy is present in the whole blood for each test chamber on a display.

2. The device of claim 1, wherein the vacuum source is configured to selectively couple to a plurality of vacuum ports (42) in the cartridge, wherein the cartridge comprises a plurality of hydrophobic filters in line with each of the vacuum ports, the hydrophobic filters configured to stop the flow of blood when each of the plurality of reagent chambers is filled.

3. The device of claim 1, further comprising a heating element (16, 42) configured to apply thermal energy to the cartridge and to the whole blood sample.

4. The device of claim 3, wherein the heating element heats the whole blood sample to a temperature between 34 degrees Celsius and 37 degrees Celsius

5. The device of claim 3, wherein the heating element heats the whole blood sample to a temperature between 30 degrees Celsius and 33 degrees Celsius.

6. The device of claim 4, wherein, in use, the whole blood sample is mixed with a reagent in each of the plurality of reagent chambers and incubated between 1 minute and 10 minutes.

7. The device of claim 6, wherein the reagents in each of the plurality of reagent chambers is isolated from its adjacent reagent chambers.

8. The device of claim 1, wherein the controller is further configured to determine a length of time to clot formation in each of the plurality of test chambers.

9. The device of claim 8, wherein the length of time is based on how long a respective sensor of the plurality of sensors that is associated with a test chamber detects movement of the spherical member.

10. The device of claim 1, wherein for each test chamber, two sensors of the plurality of sensors (54) are configured to be associated with a test chamber, optionally wherein the two sensors are configured to be adjacent to the test chamber, optionally still wherein the two sensors spaced apart and located linearly along the travel path of the spherical member (114).

11. The device of claim 10, wherein the controller is further configured to determine clot formation.

12. The device of claim 11, wherein clot formation is determined when the controller detects the absence of two peaks in each agitation cycle and is indicative that the spherical member has stopped moving within the test chamber.

13. The device of claim 11, wherein the controller is further configured to determine clot integrity, optionally:
(a) wherein a clot of high integrity is determined where neither one of the two sensors generates a signal after the clot formation; or
(b) wherein a clot of low integrity is determined where one of the two sensors continues to generate a signal indicating that the clot is allowing the spherical member to move over a limited distance; or
(c) wherein clot integrity is based on an average signal in the test chamber generated by the two sensors from the time the clot formed to the end of the test.

14. The device of claim 1, wherein the cartridge includes 18 test chambers, optionally wherein each test chamber is associated with a reagent chamber, and wherein each test chamber receives a whole blood-reagent complex from the associated reagent chamber.

15. The device of claim 1:
(a) wherein the spherical member in each test chamber passes over the sensors associated with each test chamber to generate a signal that is proportional to the viscosity of the whole blood sample within each test chamber; or
(b) wherein the controller is further configured to determine a diagnosis based on results of the coagulopathy; or
(c) wherein the controller is further configured to output the diagnosis on the display.

## Patentansprüche

1. Vorrichtung (10) zum Verarbeiten einer Kartusche (30), die eine Vollblutprobe umfasst, wobei die Vorrichtung umfasst:
eine Aussparung (18) zum Aufnehmen der Kartusche,
eine Unterdruckquelle (12), die dazu konfiguriert ist, mit der Kartusche gekoppelt zu werden,
eine Betätigungsvorrichtung (32), die dazu konfiguriert ist, mit der Kartusche verlinkt zu werden, um die Kartusche zu schütteln,
eine Steuereinheit (20),
eine Vielzahl von Sensoren (54) in Verbindung mit der Steuereinheit, wobei jeder Sensor dazu konfiguriert ist, einer Prüfkammer einer Vielzahl von Prüfkammern zugehörig zu sein, aus denen die Kartusche besteht, und
eine Vielzahl von Magneten (50, 118), wobei jeder Magnet dazu konfiguriert ist, an eine Prüfkammer der Vielzahl von Prüfkammern, aus denen die Kartusche besteht, angrenzend positioniert zu sein;
wobei die Steuereinheit im Gebrauch konfiguriert ist:
die Unterdruckquelle zu aktivieren, um die Vollblutprobe von einem Behälter in eine Vielzahl von Kanälen in der Kartusche, und danach in eine Vielzahl von Reagenskammern in der Kartusche, wo sich das Blut mit einem Reagens vermischt, und danach durch eine Vielzahl von serpentinenartigen Kanälen in eine Vielzahl von Prüfkammern in der Kartusche zu bewegen, wobei jede der Vielzahl von Prüfkammern ein kugelförmiges Element umfasst,
die Betätigungsvorrichtung zu aktivieren, um die Kartusche zu schütteln,
Signale von der Vielzahl von Sensoren zu empfangen, wobei jeder Sensor einer Prüfkammer zugehörig ist, wobei die Signale auf dem Vorhandensein des kugelförmigen Elements in einer jeweiligen Prüfkammer innerhalb eines Magnetfelds basieren, das von einem Magneten der Vielzahl von Magneten erzeugt wird,
zu bestimmen, ob Gerinnungsstörung in dem Vollblut für jede Prüfkammer vorliegt, und
einen Indikator, ob Gerinnungsstörung in dem Vollblut für jede Prüfkammer vorliegt, auf einer Anzeige auszugeben.

2. Vorrichtung nach Anspruch 1, wobei die Unterdruckquelle dazu konfiguriert ist, selektiv an eine Vielzahl von Unterdruckanschlüssen (42) in der Kartusche gekoppelt zu werden, wobei die Kartusche eine Vielzahl von wasserabweisenden Filtern in Reihe mit jedem der Unterdruckanschlüsse umfasst, wobei die wasserabweisenden Filter dazu konfiguriert sind, den Blutfluss zu stoppen, wenn jede der Reagenskammern gefüllt ist.

3. Vorrichtung nach Anspruch 1, ferner umfassend ein Heizelement (16, 42), das dazu konfiguriert ist, Wärmeenergie auf die Kartusche und auf die Vollblutprobe anzuwenden.

4. Vorrichtung nach Anspruch 3, wobei das Heizelement die Vollblutprobe auf eine Temperatur zwischen 34 Grad Celsius und 37 Grad Celsius erwärmt

5. Vorrichtung nach Anspruch 3, wobei das Heizelement die Vollblutprobe auf eine Temperatur zwischen 30 Grad Celsius und 33 Grad Celsius erwärmt.

6. Vorrichtung nach Anspruch 4, wobei die Vollblutprobe optional in jeder der Reagenskammern mit einem Reagens gemischt und zwischen 1 Minute und 10 Minuten inkubiert wird.

7. Vorrichtung nach Anspruch 6, wobei das Reagenz in jeder der Reagenskammern optional außerdem von ihren angrenzenden Reagenskammern isoliert ist.

8. Vorrichtung nach Anspruch 1, wobei die Steuereinheit ferner dazu konfiguriert ist, eine Zeitspanne bis zur Gerinnselbildung in jeder der Prüfkammern zu bestimmen.

9. Vorrichtung nach Anspruch 8, wobei die Zeitspanne darauf basiert, wie lange ein jeweiliger Sensor der Vielzahl von Sensoren, der zu einer der Prüfkammern gehört, eine Bewegung des kugelförmigen Elements erkennt.

10. Vorrichtung nach Anspruch 1, wobei für jede Prüfkammer zwei Sensoren der Vielzahl von Sensoren (54) dazu konfiguriert sind, einer Prüfkammer anzugehören, wobei die beiden Sensoren optional dazu konfiguriert sind, angrenzend an die Prüfkammer angeordnet zu sein, wobei die beiden Sensoren weiter optional beabstandet sind und sich linear entlang eines Verfahrweges des kugelförmigen Elements (114) befinden.

11. Vorrichtung nach Anspruch 10, wobei die Steuereinheit ferner dazu konfiguriert ist, Gerinnselbildung zu bestimmen.

12. Vorrichtung nach Anspruch 11, wobei eine Gerinnselbildung bestimmt wird, wenn die Steuereinheit das Fehlen von zwei Spitzen in jedem Schüttelzyklus erkennt, und angibt, dass sich das kugelförmige Element in der Prüfkammer nicht mehr bewegt.

13. Vorrichtung nach Anspruch 11, wobei die Steuereinheit ferner dazu konfiguriert ist, Gerinnselintegrität zu bestimmen.
(a) wobei ein Gerinnsel mit hoher Integrität bestimmt wird, wenn nach der Gerinnselbildung keiner der zwei Sensoren ein Signal erzeugt; oder
(b) wobei ein Gerinnsel mit geringer Integrität bestimmt wird, wenn einer der zwei Sensoren weiterhin ein Signal erzeugt, was angibt, dass das Gerinnsel ermöglicht, dass sich die Metallkugel über eine begrenzte Distanz bewegt; oder
(c) wobei eine Gerinnselintegrität auf einem Durchschnittssignal in der Prüfkammer basiert, das durch die zwei Sensoren vom Zeitpunkt der Gerinnselbildung bis zum Ende der Prüfung erzeugt wird.

14. Vorrichtung nach Anspruch 1, wobei die Kartusche 18 Prüfkammern einschließt, wobei jede Prüfkammer optional zu einer Reagenskammer gehört, und wobei jede Prüfkammer einen Vollblut-Reagens-Komplex von der zugehörigen Reagenskammer empfängt.

15. Vorrichtung nach Anspruch 1:
(a) wobei das kugelförmige Element in jeder Prüfkammer über den Sensor läuft, der zu jeder Prüfkammer gehört, um ein Signal zu erzeugen, das proportional zur Viskosität der Vollblutprobe in jeder Prüfkammer ist; oder
(b) wobei die Steuereinheit ferner dazu konfiguriert ist, basierend auf Ergebnissen der Gerinnungsstörung eine Diagnose zu bestimmen; oder
(c) wobei die Steuereinheit optional ferner dazu konfiguriert ist, die Diagnose auf der Anzeige auszugeben.

## Revendications

1. Dispositif (10) destiné au traitement d'une cartouche (30) contenant un échantillon de sang total, le dispositif comprenant :
un logement (18) destiné à recevoir la cartouche,
une source de vide (12) configurée pour être raccordée à la cartouche,
un actionneur (32) configuré pour être relié à la cartouche afin de l'agiter,
un dispositif de commande (20),
une pluralité de capteurs (54) en communication avec le dispositif de commande, chaque capteur étant configuré pour être associé à l'une des chambres de test comprises dans la cartouche, et
une pluralité d'aimants (50, 118), dans lequel chaque aimant est configuré pour être placé à proximité d'une chambre de test parmi la pluralité de chambres de test que comprend la cartouche ;
dans lequel, en utilisation, le dispositif de commande est configuré pour :
activer la source de vide pour transférer l'échantillon de sang total d'un récipient vers une pluralité de canaux de la cartouche, puis vers plusieurs chambres à réactif dans la cartouche où le sang se mélange à un réactif, et ensuite, à travers plusieurs canaux en forme de serpentin, vers plusieurs chambres de test dans la cartouche, chacune de la pluralité des chambres de test comprenant un élément sphérique,
activer l'actionneur pour agiter la cartouche,
recevoir des signaux provenant de la pluralité de capteurs, chaque capteur étant associé à une chambre de test, dans laquelle lesdits signaux sont basés sur la présence de l'élément sphérique dans une chambre de test respective au sein d'un champ magnétique généré par un aimant de la pluralité d'aimants,
déterminer s'il existe une coagulopathie dans le sang total pour chaque chambre de test, et
afficher un indicateur précisant si une coagulopathie est présente dans le sang total pour chaque chambre de test.

2. Dispositif selon la revendication 1, dans lequel la source de vide est configurée pour se raccorder de manière sélective à une pluralité d'orifices de vide (42) dans la cartouche, dans lequel la cartouche comprend une pluralité de filtres hydrophobes disposés en série avec chacun des orifices de vide, les filtres hydrophobes étant configurés pour interrompre l'écoulement du sang lorsque chacune des chambres à réactif de la pluralité est remplie.

3. Dispositif selon la revendication 1, comprenant en outre un élément chauffant (16, 42) configuré pour appliquer de l'énergie thermique à la cartouche et à l'échantillon de sang total.

4. Dispositif selon la revendication 3, dans lequel l'élément chauffant chauffe l'échantillon de sang total à une température comprise entre 34 °C et 37 °C

5. Dispositif selon la revendication 3, dans lequel l'élément chauffant chauffe l'échantillon de sang total à une température comprise entre 30 °C et 33 °C

6. Dispositif selon la revendication 4, dans lequel, lors de l'utilisation, l'échantillon de sang total est mélangé à un réactif dans chacune des différentes chambres à réactif, puis incubé pendant une durée comprise entre 1 et 10 minutes.

7. Dispositif selon la revendication 6, dans lequel les réactifs contenus dans chacune des différentes chambres à réactifs sont isolés de ceux des chambres à réactifs adjacentes.

8. Dispositif selon la revendication 1, dans lequel le dispositif de commande est en outre configuré pour déterminer le temps nécessaire à la formation d'un caillot dans chacune des différentes chambres de test.

9. Dispositif selon la revendication 8, dans lequel la durée est déterminée en fonction du temps pendant lequel un capteur donné parmi la pluralité de capteurs, associé à une chambre de test, détecte le mouvement de l'élément sphérique.

10. Dispositif selon la revendication 1, dans lequel, pour chaque chambre de test, deux capteurs parmi la pluralité de capteurs (54) sont configurés pour être associés à une chambre de test, dans lequel, optionnellement, les deux capteurs peuvent être configurés pour être adjacents à la chambre de test, et optionnellement encore, dans lequel les deux capteurs peuvent également être espacés l'un de l'autre et disposés de manière linéaire le long du trajet de déplacement de l'élément sphérique (114).

11. Dispositif selon la revendication 10, dans lequel le dispositif de commande est en outre configuré pour déterminer la formation d'un caillot.

12. Dispositif selon la revendication 11, dans lequel la formation d'un caillot est déterminée lorsque le dispositif de commande détecte l'absence de deux pics au cours de chaque cycle d'agitation, ce qui indique que l'élément sphérique a cessé de se déplacer à l'intérieur de la chambre de test.

13. Dispositif selon la revendication 11, dans lequel le dispositif de commande est en outre configuré pour déterminer la formation d'un caillot, optionnellement :
(a) dans lequel on détermine la présence d'un caillot de haute intégrité lorsque aucun des deux capteurs ne génère de signal après la formation du caillot; ou
(b) dans lequel un caillot de faible intégrité est détecté lorsque l'un des deux capteurs continue à générer un signal indiquant que le caillot permet à l'élément sphérique de se déplacer sur une distance limitée ; ou
(c) dans lequel l'intégrité du caillot est déterminée à partir d'un signal moyen dans la chambre de test généré par les deux capteurs, depuis le moment où le caillot s'est formé jusqu'à la fin du test.

14. Dispositif selon la revendication 1, dans lequel la cartouche inclut 18 chambres de test, dans lequel optionnellement, chaque chambre de test peut être associée à une chambre à réactif, et dans lequel chaque chambre de test reçoit un complexe sang total-réactif provenant de la chambre à réactif associée.

15. Dispositif selon la revendication 1 :
(a) dans lequel l'élément sphérique situé dans chaque chambre de test passe au-dessus des capteurs associés à chaque chambre de test afin de générer un signal qui est proportionnel à la viscosité de l'échantillon de sang total contenu dans chaque chambre de test ; ou
(b) dans lequel le dispositif de commande est en outre configuré pour déterminer un diagnostic fondé sur les résultats de l'examen de la coagulation ; ou
(c) dans lequel le dispositif de commande est en outre configuré pour afficher le diagnostic à l'écran.
